# EUROPEAN PATENT SPECIFICATION

(11) **EP 2 682 121 B1**
(45) Date of publication and mention of the grant of the patent: **14.03.2018**
(21) Application number: 12751830.6
(22) Date of filing: 31.01.2012
(51) Int. Cl.: A61K 31/496, A61P 37/06, A61P 29/00, A61P 37/02, A61P 7/00

(54) **PHARMACEUTICAL USE OF AMINOTHIAZOLE MYD88 SPECIFICITY INHIBITOR**
PHARMAZEUTISCHE VERWENDUNG VON AMINOTHIAZOL-MYD88-SPEZIFITÄTSHEMMERN
UTILISATION PHARMACEUTIQUE DE L'INHIBITEUR DE SPÉCIFICITÉ D'AMINOTHIAZOLE MYD88

(30) Priority: 02.03.2011 CN 201110049579
(43) Date of publication of application: 08.01.2014
(73) Proprietor: Wuhan Innamune Pharmaceutical Co., Ltd, Wuhan, Hubei (CN)
(72) Inventor: ZHOU, Ping, Wuhan Hubei 430030 (CN); JIANG, Fengchao, Wuhan Hubei 430030 (CN)
(74) Representative: Zeuner Summerer Stütz
(86) International application number: PCT/CN2012/070808
(87) International publication number: WO 2012/116585

(56) References cited:
- CN-A- 102 166 214
- CN-A- 102 336 720
- PAPADOPOULOU C ET AL: "Synthesis and biological evaluation of new thiazolyl/benzothiazolyl-amides, derivatives of 4-phenyl-piperazine", IL FARMACO, ELSEVIER FRANCE * EDITIONS SCIENTIFIQUES ET MEDICALES, IT, vol. 60, no. 11-12, 1 November 2005 (2005-11-01), pages 969-973, XP027697620, ISSN: 0014-827X [retrieved on 2005-11-01]
- WEN-TING ZHANG ET AL: "Design, synthesis, and cytoprotective effect of 2-aminothiazole analogues as potent poly(ADP-ribose) polymerase-1 inhibitors", JOURNAL OF MEDICINAL CHEMISTRY, AMERICAN CHEMICAL SOCIETY, US, vol. 52, no. 3, 12 February 2009 (2009-02-12), pages 718-725, XP002640248, ISSN: 0022-2623, DOI: 10.1021/JM800902T [retrieved on 2009-01-06]
- PAPADOPOULOU, C. ET AL.: 'Synthesis and biological evaluation of new thiazolyl/benzothiazolyl-amides, derivatives of 4-phenyl-piperazine.' IL FARMACO. vol. 60, 2005, pages 969 - 973, XP027697620

## Description

### FIELD OF THE INVENTION

The invention relates to the use of aminothiazole-based specific inhibitor of MyD88 as an immunomodulator in fields of medicine and scientific research, and more particularly to the use thereof in treatment of anti-transplant rejection, anti-autoimmune disease, anti-ischemia-reperfusion injury, anti-chronic inflammation, and anti-endotoxaemia.

### BACKGROUND OF THE INVENTION

It is known that inhibitory regulation of organism immune system is a key element to treat a plurality of diseases, such as, organ transplantation rejection, autoimmune disease, chronic inflammatory diseases, ischemia-reperfusion injury, and the like. The regulation of the immune system can be started from different aspects. As a research hotspot, innate immune response has been an excellent direction for realizing the immunologic suppression.

Organism immune response includes innate immunity and acquired immunity. Because of the excellent specific recognizing ability and highly effective response results, the acquired immunity has always been a primary study subject and intervening target. The traditional immune response has been recognized as that a first stimulation signal and a second stimulation signal of the required immune system activate NF-κB, the activated NF-κB enters nucleus and initiates the transcription to allow the cell to synthesize and secrete various inflammatory factors and stimulate a series of immune responses. The current anti-rejection drugs act on the required immune system. Innate immunity has been considered as an innate protective barrier of the organism against the infection of virus and bacteria and the invasion of alien organisms. Most studies of the recent years have found that innate immune system plays a very important role in the transplantation immunity, autoimmune diseases, ischemic injury, and the like. For innate immune system, tool-like Receptor (TLR) plays a primary role and has been a study focus. At least 14 subscriber sets of TLR have been found, these subscriber sets of TLR are mostly distributed on APC immune cells and the like. All subscriber sets have to use myeloid-differentiation protein 88 (MyD88) to transmit signals except for TLR3. A large number of studies have found that various endogenous and exogenous risk factors activate each TLR of the innate immune system and stimulate signals to transmit through key molecule of MyD88, and finally activate NF-κB. Subsequent immune response is the same as the former.

In summary, MyD88 is a key molecule node in the innate immunity. To block MyD88 is to block the main reaction of the innate immune system, thereby producing corresponding immunosuppressive effect. Providing that a drug is capable of intervening and blocking MyD88, primary signals of the TLR pathway can be blocked by such a drug, thereby forming a series of immune regulations; this is one of the best scheme for immunotherapy.

Many papers have published and demonstrated the importance of MyD88 and the treatment effect, but have not found a drug for inhibiting MyD88. Other methods, such as gene knockout for interveing MyD88 can not be applied in clinic treatment.

Inventor has carried out a series of preliminary studies on TLR/MyD88, and has verified the important role of TLR in transplantation immunity, and proved that the block of MyD88 molecule is capable of inducing and maintaining the tolerence of transplantation immunity. In later studies, the inventor co-operated with pharmaceutics teams, synthesized and repeatedly screened a kind of specific inhibitors of MyD88, that is, an aminothiazole-based small molecular compounds (labeled as TJ-M2010). These small molecular compounds have active sites that are capable of specifically combining with key active sites of MyD88 molecule, thereby being able to form competitive combination and inhibit corresponding signal transmission of MyD88. Thus, the specific inhibitor TJ-M2010 of MyD88 is applicable in anti-rejection, anti-autoimmune disease, anti-ischemia-reperfusion injury, anti-chronic inflammation, and anti-endotoxemia. Thus, the applications of these small molecules are started, and it is possible to develop new medicines containing these small molecules to treat various related innate immune diseases.

### SUMMARY OF THE INVENTION

It is one objective of the invention to provide a group of aminothiazole-based small molecular compounds functioning as specific inhibitors of MyD88 to suppress MyD88 molecules in innate immune system to treat a variety of immune diseases. A basis for achieving the specific technical scheme of the invention is that because of the suppressive effect on MyD88 molecules in innate immune system, aminothiazole-based MyD88 molecular analogues can be used in treatment of anti-rejection, anti-autoimmune disease, anti- ischemia-reperfusion injury, anti-chronic inflammation, and anti-endotoxemia.

MyD88 protein herein referred comprises two structural domains: toll/IL-1 receptor domain (TIR) and death domain (DD). The TIR domain is a material basis for the homologous dimerization of MyDD88 and the activation of downstream IRAK1 or IRAK4. From analysis of topological structure of the TIR domain of MyD88, a group of specific inhibitors TJ-M2010 of MyD88 are synthesized. TJ-M2010 are capable of specifically combining with the TIR domain of MyD88 and intervening the function of the TIR domain, so that the homologous dimerization of MyD88 is inhibited, the MyD88 is prevented from activation, the MyD88 transduction pathway is blocked, NF-κB is unable to be activated, and the inflammation reaction is blocked. Thus, inhibitors TJ-M2010 of MyD88 are very important in treatment of related inflammation and immune diseases.

An aminothiazole-based specific inhibitor of MyD88 (myeloid differentiation protein 88), the specific inhibitor acting as an immunomodulator and being represented by the following formulas:
TJ-M2010-1 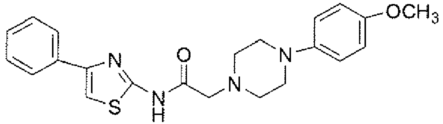 2-(4-(4-methoxyphenyl)piperazin-1-yl)-N-(4-phenyl-thiazole-2-yl)acetamide
TJ-M2010-2 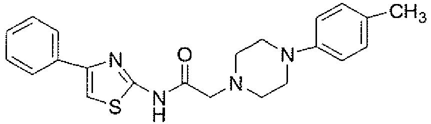 2-(4-(p-tolyl)piperazin-1-yl)-N-(4-phenyl-thiazole-2-yl)acetamide
TJ-M2010-3 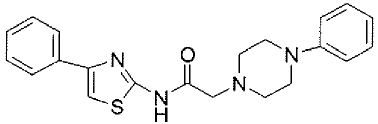 2-(4-phenyl-piperazin-1-yl)-N-(4-phenyl-thiazole-2-yl)acetamide
TJ-M2010-4 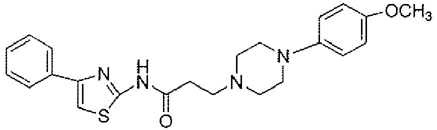 3-(4-(4-methoxyphenyl)piperazin-1-yl)-N-(4-phenyl-thiazole-2-yl)-propionamide

The aminothiazole-based specific inhibitor of MyD88 has a small molecule, stable structure, and is capable of penetrating the cell membrane and is applicable in vitro and in vivo.

The aminothiazole-based specific inhibitor of MyD88 is used as an NF-κB inhibitor for preparation of drugs comprising an immunosuppressive agent.

The aminothiazole-based specific inhibitor of MyD88 is used as immunomodulator for reducing rejections after organ transplantation and for inducing and maintaining immunologic tolerance.

The aminothiazole-based specific inhibitor of MyD88 is used as an immunomodulator for the treatment of chronic inflammatory diseases, such as chronic inflammatory bowel disease and asthma.

The aminothiazole-based specific inhibitor of MyD88 is used as an immunomodulator for treatment of autoimmune diseases, such as type I diabetes, multiple sclerosis, and lupus erythematosus.

The aminothiazole-based specific inhibitor of MyD88 is used as an immunomodulator for treatment of ischemia-reperfusion injuries, such as preventing ischemia-reperfusion injuries after myocardial infarction, replantation, and transplantation; and being used in preparations of organ preservation solution and cell preservation solution.

The aminothiazole-based specific inhibitor of MyD88 is used as an immunomodulator for prevention of endotoxemia and sepsis.

The invention is advantageous in that a group of new compounds TJ-M2010 are applied in MyD88 specific inhibition experiments, and have obvious effects in anti-rejection, anti-autoimmune disease, anti- ischemia-reperfusion injury, anti-chronic inflammation, and anti-endotoxemia. The new compounds TJ-M2010 are effective immunosuppressants, immunologic tolerance inducing agents, immunologic tolerance maintaining agents, anti-inflammatory drugs, and immunomodulators. These new compounds are able to effectively inhibit the expression of DC80 and CD86 and inhibit the mature of (dendritic cells) DC cells. The mature of DC cells has been proved to be one of critical steps for a variety of autoimmune diseases, such as autoimmune myocarditis, experimentally autoimmune grapes inflammation, type I diabetes, multiple sclerosis, lupus erythematosus, and the like. Inhibitors TJ-M2010 of MyD88 can be used in treatment of such diseases. The blockage of the MyD88 pathway has obviously protective function in ischemia-reperfusion injury, so that inhibitors TJ-M2010 of MyD88 can be used for preventing ischemia-reperfusion injuries after myocardial infarction, replantation, and transplantation, and can be used in organ preservation solution, cell preservation solution, and other aspects. Results of experiment in vitro shows that inhibitors TJ-M2010 of MyD88 effectively lower the inflammatory factors in an implant by inhibiting the pathway of MyD88, which indicates that inhibitors of MyD88 have a close relationship with inflammatory factors and the inhibitors of MyD88 are possible to be an effective means to treat various inflammatory diseases.

### BRIEF DESCRIPTION OF THE DRAWINGS

FIG. **1** shows a survival curve of a cardiac allograft;
FIG. **2** shows a survival curve of a skin graft;
FIG. **3** is diagrams show that TJ-M2010 reduces the activation of T cells and inhibits the increase of stimulus CD80 stimulated by LPS and CpG;
FIG **4** shows a curve chart that TJ-M2010 reduces the incidence of diabetes;
FIG. **5** shows a block diagram that TJ-M2010 affects the lymphocyte subset of a transplant recipient;
FIG **6** shows that TJ-M2010 improves the survival rate of mouse kidney IRI;
FIG **7** shows that TJ-M2010 protects the renal function of mouse kidney IRI;

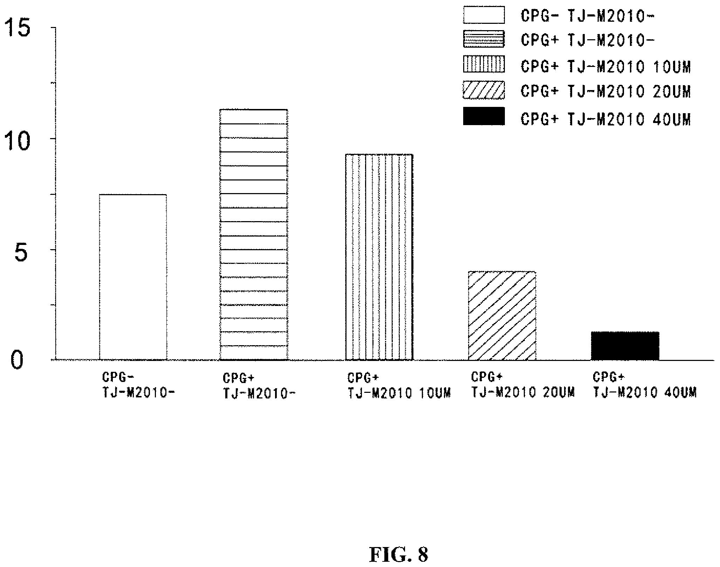

FIG. **8** shows a T cell proliferation chart when TJ-M2010 weakens CpG to stimulate the activation of DC;
FIG **9** shows that TJ-M2010 reduces inflammatory factors in an implant by a real-time quantitative PCR analysis;
FIG **10** shows that TJ-M2010 reduces the effusion of inflammatory cells in a pneumonia model;
FIG. **11** shows that TJ-M2010 reduces the effusion of inflammatory factors in a pneumonia model; and
FIG. **12** shows a survival curve of lethal experiments caused by endotoxin and sepsis.

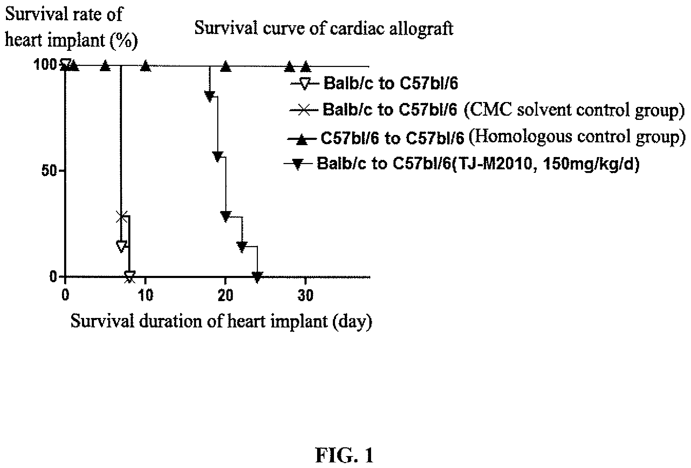

In FIG. **1****,** Balb/c and C57bl/6 are two species of mouse. Heart transplantations from Balb/c to C57bl/6 are divided into a common control group, a CMC solvent control group, and an experimental group. Heart transplantation from C57bl/6 to C57bl/6 is a homologous control group.

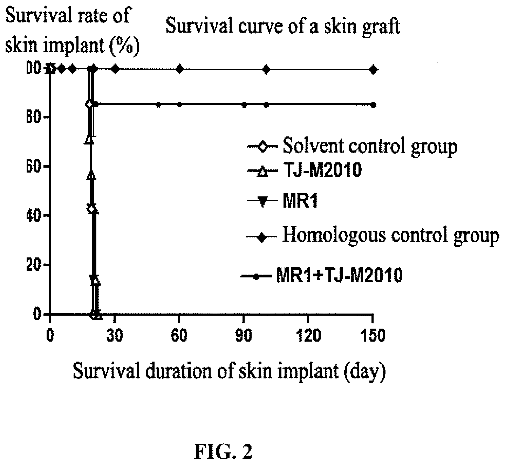

FIG. **2** comprises allogeneic skin graft control groups (it it reported the rejection time is between 8 and 10 days), a sole TJ-M2010 group, a sole MR1 group, and a mixed reagent group (TJ-M2010+MR1).

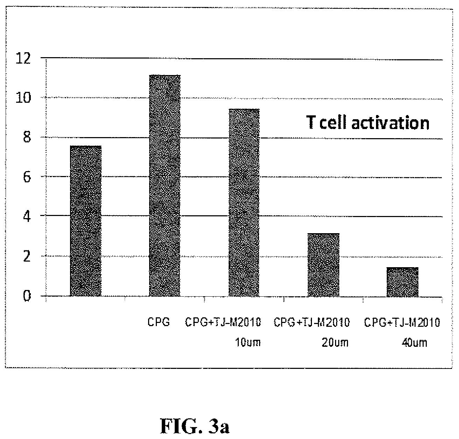

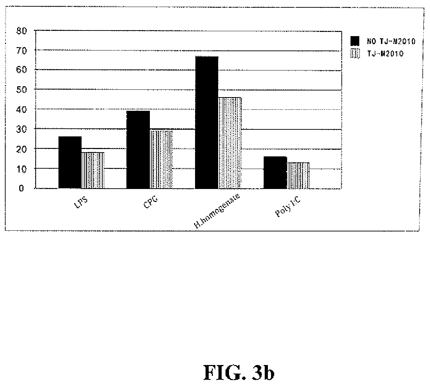

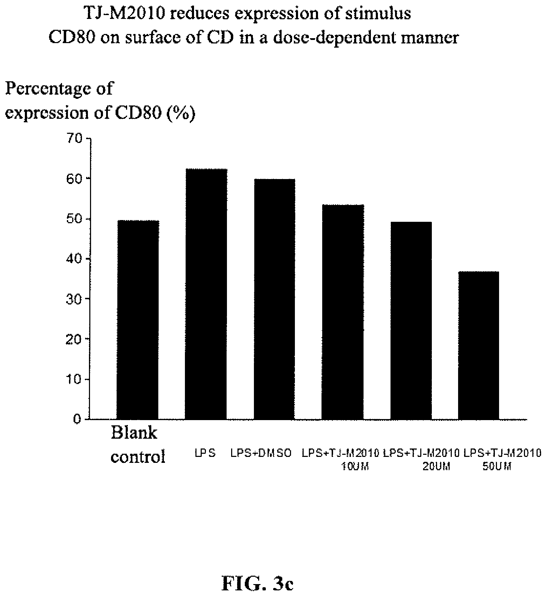

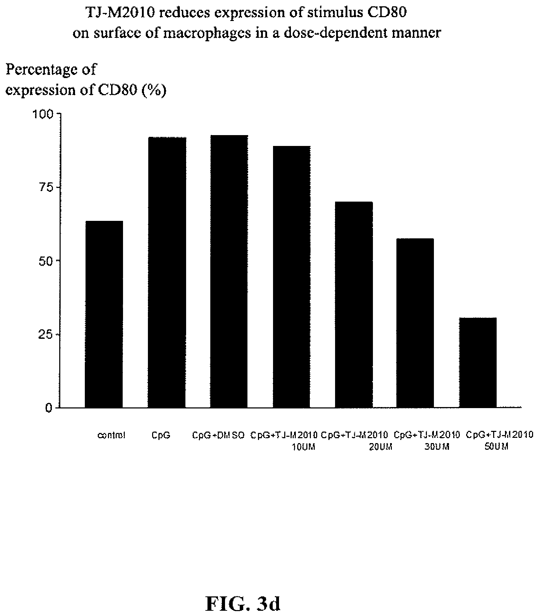

FIG. **3(a)** show that TJ-M2010 reduces the activation of T cells in a dose-dependent manner. FIG. **3(b)** show that TJ-M2010 inhibits the increase of stimulus CD80 stimulated by LPS, CpG, and homogenate of myocardial tissues. FIG. **3(c)** shows that TJ-M2010 reduces the expression of stimulus CD80 on the surface of CD in a dose-dependent manner. FIG. **3(d)** shows that TJ-M2010 reduces the expression of stimulus CD80 on the surface of macrophages in a dose-dependent manner.

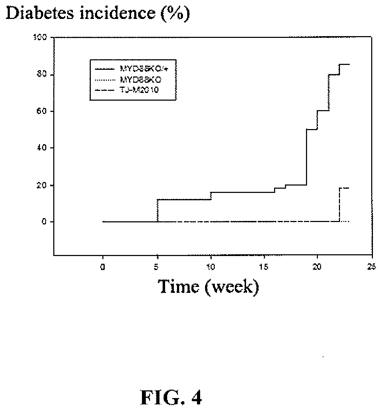

In FIG. **4****,** the experimental group comprises a group of MyD88KO NOD mouse, a group of MyD88KO/+NOD mouse, and a group of TJ-M2010 + NOD mouse.

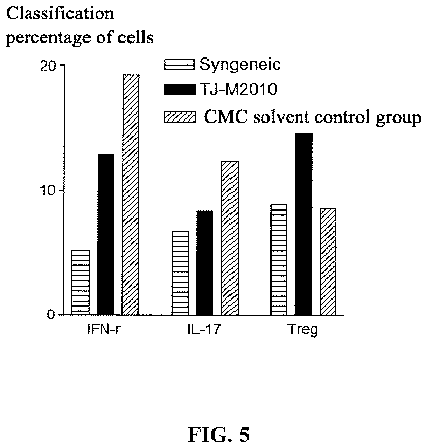

FIG. **5** shows spleen lymphocyte subsets in different recipient groups (syngeneic group, treatment group, and control group), and the result shows that the cell percentages of CD4⁺CD25⁺Foxp3⁺T in the TJ-M2010 treatment group increase significantly.

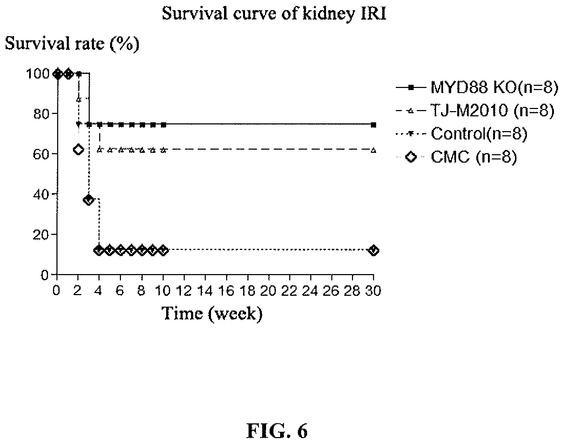

In FIG. **6****,** the experimental group comprises MYD88 KO, TJ-M2010, CMC, and a control group, each having 8 mice

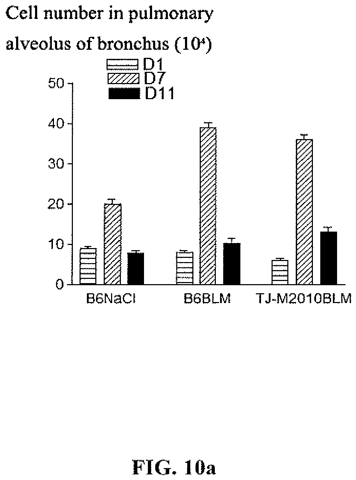

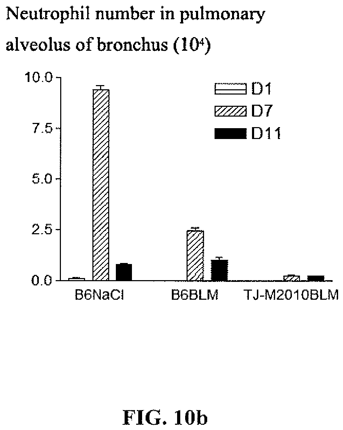

FIG **10(a)** and FIG. **10(b)** represent cell number and neutrophil number in pulmonary alveolus of bronchus, respectively.

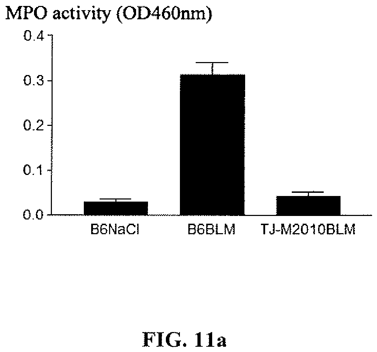

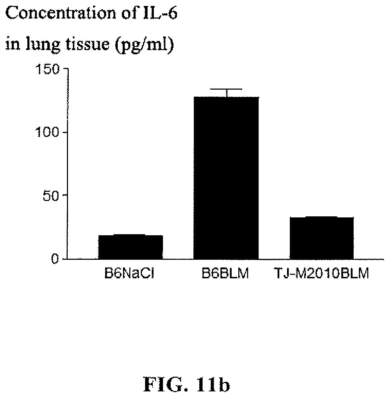

FIG. **11(a)** represents myeloperoxidase (MPO) activity in lung tissues, and FIG. **11(b)** represents the concentration of interleukin -6 in lung tissues.

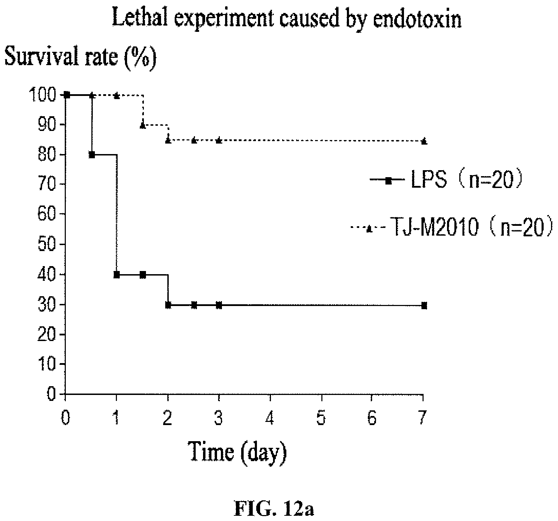

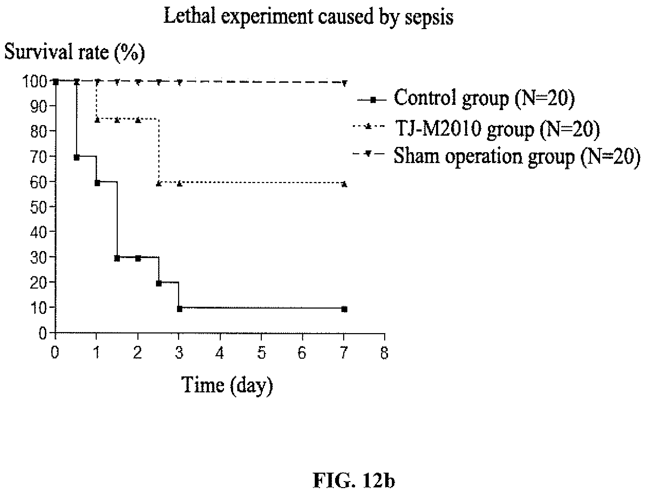

FIG. **12(a)** represents a survival curve of a lethal experiment caused by endotoxin; and FIG. **12(b)** represents a survival curve of a lethal experiment caused by sepsis.

### DETAILED DESCRIPTION OF THE EMBODIMENTS

### Example 1 Use of TJ-M2010 in anti-transplantation rejection and induction of immnologic tolerance

1) TJ-M2010 was used in heart transplantation model of mice.
   The experiment had the following four groups:
   Blank control group (without any treatment except for heart transplantation); CMC group (vehicle control group), isograft group (theoretically, transplantation between two genetically identical individuals has no rejection, and this group can be survival for a long term), and TJ-M2010 administered group (effect detection group). Specific treatment of each group was as follows:
      The blank control group: hearts of the Balb/c mice were grafted to abdominal cavities of C57bl/6 mice, respectively;
      The CMC control group: hearts of the Balb/c mice were grafted to abdominal cavities of C57bl/6 mice, respectively; and between 0 and 6 days before the heart transplantation, 200 µL of a carboxymethylcellulose sodium solution (0.5% CMC) not containing TJ-M2010 was administered by intraperitoneal injection;
      The isograft group: hearts of C57bl/6 mice were grafted to abdominal cavities of genetically identical C57bl/6 mice, respectively; no special treatment was performed after the surgery; and
      TJ-M2010 administered group: hearts of the Balb/c mice were grafted to abdominal cavities of C57bl/6 mice, respectively; and between 0 and 6 days before heart transplantation, TJ-M2010 dissolved in CMC was administered by intraperitoneal injection, a dose thereof was 150 mg/kg.

   Experiment results were shown in a survival curve chart (as shown in FIG. **1**). Rejection duration of the blank control group was basically the same as that in reported literatures, about eight days. The CMC control group had no difference. The isograft group survived for a long term, and average survival duration of the heart implant of the TJ-M2010 administered group was approximately 20 days, which was obviously longer than control groups.
2) Inhibitor of TJ-M2010 combined with co-stimulatory molecule (Anti-CD 154 mAb (MR1)) was used in skin transplantation model of mice.

The experiment had five groups: allogeneic skin transplantation control group (rejection duration reported in literatures was between eight and ten days); syngeneic skin transplantation control group; individually TJ-M2010 administered group; individually MR1 administered group; and TJ-M2010+MR1 administered group.

### Specific treatments were as follows:

Allogeneic skin transplantation control group: skins of Balb/c mice were transplanted to backs of C57bl/6 mice, respectively; 0.5% CMC was administered by abdominal injection on the 0-3^{rd}, 5^{th}, 7^{th}, 9^{th}, 11^{th}, 13^{th}, and 15^{th} days after the surgery, and a dose thereof was 200 µL each day;
Syngeneic skin transplantation control group: skins of C57bl/6 mice were transplanted to backs of C57bl/6 mice, respectively; no special treatment was performed after the surgery;
Individually TJ-M2010 administered group: skins of Balb/c mice were transplanted to backs of C57bl/6 mice, respectively; TJ-M2010 dissolved in 0.5% CMC was administered by abdominal injection on the 0-3^{rd}, 5^{th}, 7^{th}, 9^{th}, 11^{th}, 13^{th}, and 15^{th} days after the surgery, and a dose thereof was 150 mg/kg/d;
Individually MR1 administered group: skins of Balb/c mice were transplanted to backs of C57bl/6 mice, respectively; MR1 was administered by abdominal injection on the 0, 1^{st}, 3^{rd}, 5^{th}, 7^{th}, 9^{th}, 11^{th}, 13^{th}, and 15^{th} days after the surgery, and a dose thereof was 200 µL each day; and
TJ-M2010+MR1 administered group: skins of Balb/c mice were transplanted to backs of C57bl/6 mice, respectively; TJ-M2010 dissolved in 0.5% CMC was administered by abdominal injection on the 0-3^{rd}, 5^{th}, 7^{th}, 9^{th}, 11^{th}, 13^{th}, and 15^{th} days after the surgery, and a dose thereof was 150 mg/kg/d; meanwhile, MR1 was administered by abdominal injection on the 0-3^{rd}, 5^{th}, 7^{th}, 9^{th}, 11^{th}, 13^{th}, and 15^{th} days after the surgery, and a dose thereof was 200 µL each day.

Experiment results were shown in a survival curve chart (as shown in FIG 2). Rejection duration of the skin implant in the allogeneic skin transplantation control group was basically the same as that in reported literatures, about ten days.

Rejection durations of the skin implant of the individually TJ-M2010 administered group and the individually MR1 administered group were approximately ten days, and had no statistic difference. Survival duration of the skin implant the TJ-M2010+MR1 administered group after the skin transplantation was approximately 150 days (literatures had referred that survival duration longer than 100 days indicated immunologic tolerance).

Thus, individually use of TJ-M2010 and MR1 had no significant effects on immunologic tolerance induction of the skin implant; while combined use of TJ-M2010 and MR1 had significant effect, and was able to allow the skin implant that were difficult to induce the immunologic tolerance to survive for a long term.

From the experiment results, it was known that, Inhibitor of MyD88 had an obvious effect on the anti-rejection after transplantation and induction of immunologic tolerance, and was act as a special immunosuppressive agent, immunologic tolerance inducing agent (a short period of administration contributed a long term survival to a transplanted group that were difficult to induce the immunologic tolerance of the skin implant), or immunologic tolerance maintaining agent (such as tolerance against the rejection caused by virus infection). The special function thereof cannot be substituted by the currently used immunosuppressive agent.

### Example 2 Use of inhibitor of MyD88 in treatment of autoimmune diseases

In vitro experiment - results from flow cytometry proved that inhibitors of MyD88 were capable of inhibiting the mature of DC cells for treating autoimmune diseases.

In vitro experiment comprised the following steps: 1. TJ-M201020 was applied to bone marrow cell from BALB/c mice. Membrane of the marrow cells was broken. The marrow cells were then cultivated in a RPMI1640 medium (added with GM-CSF10 ng/mL, IL-4 10 ng/mL), a concentration of the marrow cells was controlled at 2×106/mL.
2. Cells were cultivated for 48 h, and suspended cells were removed. On a 6^{th} day, suspended cells and semi-adherent cells were collected.
3. DC cells were added with 50 mM of TJ-M201020 and cultivated for 1h. The medium was then added with a supernatent of necrotic myocardium, LPS (200 ng/mL), Poly I:C (20 mg/mL), and CpG (10 mg/mL) and cultrued for 12 h.
4. Flow antibodies FITC-labeled anti-CD80, CD86 were added for testing.

TJ-M201020 inhibited the increase of the co-stimulatory molecules CD80 in RAW264.7 cells cause by TLR stimulus (LPS, CpG). Thus, TJ-M201020 effectively blocked the TLR signaling pathway and inhibited the immune response of the cell.

Experiment processes of FIG. **3(c)** and FIG. **3(c)** were as follows:
Raw264.7: 48-well plate, cells number was 9*105/well. Each well was added with 1 mL of culture system. Different concentrations of TJ-M201020 were added, and cells were pre-incubated for 2 h. CPG was then added. A final concentration was 40 µg/mL. Cells were incubated for 12 h at a temperature of 37°C in a CO₂ incubator. Flow antibodies FITC-labeled anti-CD80 and CD86 were added for testing.

DC: 48-well plate, cells number was 1*106/well. Each well was added with 1mL of culture system. Different concentrations of TJ-M201020 were added, and cells were pre-incubated for 2 h. LPS was then added. A final concentration was 1µg/mL. Cells were incubated for 12 h at a temperature of 37°C in a CO₂ incubator. Flow antibodies FITC-labeled anti-CD80 and CD86 were added for testing. From (b) and (c) of FIG. 3, it was known that TJ-M201020 had an inhibition effect on the expression of DC cells and macrophage cell surface CD80 correlated to a certain range of concentration.

FIG. **3** shows inhibition of TJ-M2010 against the increase of co-stimulatory molecules CD80/CD86 activated by LPS and CpG

The above test results indicated that MyD88 was capable of lowering the expression of CD80 and inhibiting the mature of DC cells. The mature of DC cells had been proved to be one of the critical steps resulting in autoimmune cardiomyopathy, experimental autoimmune inflammatory grapes, type I diabetes, multiple sclerosis, lupus erythematosus. Thus, MyD88 was capable of treating these diseases.

In vivo experiment - influence of MyD88-/- and TJ-M201020 provided in the invention on the model building of type I diabetes.

In vivo experiment comprised the following steps:
1. Experimental groups: MyD88KO NOD mice, MyD88KO/+NOD mice, TJ-M201002 administered NOD mice.
2. TJ-M201002 administered NOD mice: antigen was injected one day before, TJ-M2010 dissoved in 0.5% CMC was respectively intraperitoneally injected on a 0-3^{th} day, a 5^{th} day, a 7^{th} day, a 9^{th} day, a 11^{th} day, a 13^{th} day, a 15^{th} day, a dose thereof was 150 mg/kg/d.
3. Each group was injected with mycobacterial antigen and continuously monitored the concentration thereof.
4. Each group was feed for 30 weeks at a clear grade. After that, venous blood in cauda was collected on a non-empty stomach and blood glucose was continuously tested for twice. The diabetes modeling standard was that both blood glucose ≥22 mmol/L.

An incidence curve chart of type I diabetes was shown in FIG. **4****.**

The results showed that for MyD88KO heterozygous group, the incidence of the type I diabetes increased with the increase of the time. The MyD88KO homozygous group had no incidence of the type I diabetes. The incidence of the type I diabetes of TJ-M201002 group was equivalent to that of the MyD88KO homozygous group. Thus, MyD88 pathway had a close relationship with type I diabetes. To blockage of the MyD88 pathway was to decrease the incidence of the diabetes, so that the small molecule inhibitor of MyD88 TJ-M2010 was effective in treatment of type I diabetes.

### Example 3 Use of inhibitors of MyD88 in prevention and treatment of ischemia-reperfusion injuries

In vitro experiment: analysis of lymphocyte subsets in spleen of recipient stimulated by (syngeneic and allogeneic) antigens and analysis of CD4+CD25+Foxp3+T cells proportion inside the body of recipient administered with TJ-M2010 by using flow cytometry

In vitro experiment comprised the following steps:
1. Spleens of recipients of different groups (syngeneic and allogeneic) were ground to separate lymphocytes;
2. Flow antibody APC-labeled IFN-γ, APC-labeled IL-17, APC-labeled CD25, PE-labeled Foxp3 were added in the flow cytometry.
3. Lymphocyte subsets in spleen of recipient stimulated by (syngeneic and allogeneic) antigens and CD4+CD25+Foxp3+T cells proportion inside the body of recipient administered with TJ-M2010 were analyzed by using the flow cytometry.

As shown in FIG. **5****,** CD4+CD25+Foxp3+T cells proportion inside the body of recipient administered with TJ-M2010 were obviously increased, while the level of IFN-γ and IL-17 were significantly lower than that of the CMC control group.

After being administered with Inhibitor of MyD88 TJ-M2010, analyses of lymphocyte subsets in spleen of recipient stimulated by (syngeneic and allogeneic) antigens and CD4+CD25+Foxp3+T cells proportion represented that the use of the TJ-M2010 was to increase the CD4+CD25+Foxp3+T cells proportion to change the immunologic tolerance state of the recipient mice. A large number of literatures showed that because of the immunosuppressive function, regulatory T cells were able to regulate the development of the inflammation, the release of inflammatory factors and pro-inflammatory factors, and the crosslinking of ischemia-reperfusion cytokines, thereby resulting in injuries. Thus, the use of the TJ-M2010 was able to inhibit TLR signals, prevent the NF-κB from activation, and lower the expression of inflammatory factors (IFN-γ and IL-17) to alleviate the injuries.

In vivo experiment: blocking the MyD88 pathway to alleviate ischemia-reperfusion injuries of kidney

In vivo experiment comprised the following steps:
1. The experiment had the following groups: common C57bl/6 control group, CMC vehicle group, MYD88KO group, and TJ-M2010 group. Each group had eight mice treated by ischemia-reperfusion: each mouse was administered with anesthetics, a left kidney was blocked by a vascular clamp, and was preserved in a calorstat at a temperature of 31°C for 80 min; thereafter, the vascular clamp was removed and a right kidney was removed, the abdomen was then sutured. Blood was collected in 24 h for BUN and Cr detections.
2. TJ-M2010 group and CMC group: TJ-M2010 dissolved in 0.5% CMC was administered by intraperitoneal injection on the day of surgery and one day before the surgery, respectively, a dose thereof was 150 mg/kg/d; CMC group was administered with 200 µL of the 0.5% CMC solution.
3. The survival duration of the mice was observed, and a survival curve was charted. Blood samples were collected for BUN and Cr detections.
4. Results showed that the TJ-M2010 significantly increased the survival rate of the mice after kidney IRE, and had a good protective effect on the function of the kidney.

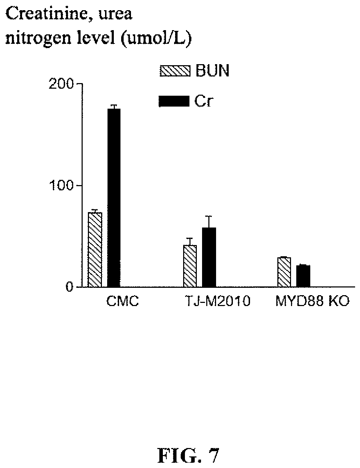

### Results were shown in FIGS. 6 and 7.

The blockage of the MyD88 pathway had obviously protective function in ischemia-reperfusion injury, so that Inhibitors TJ-M2010 of MyD88 was applicable in preventing the implant from ischemia-reperfusion injuries after myocardial infarction, replantation, and transplantation; and in organ preservation solution, cell preservation solution, and other aspects.

### Example 4 Use of inhibitor of MyD88 in treatment of chronic inflammatory diseases

In vitro experiment: real-time quantitative PCR analysis of T cell proliferation and inflammatory factors in an implant under the action of TJ-M2010 in weakening the activation of DC cells by suppressing CpG

The in vitro experiment comprised the following steps:
1. Femurs of Bal b/c mice were provided, and bone marrow cells were separated and added with GMS-CSF and IL-4 cytokine for culturing bone marrow-derived DC.
2. bone marrow-derived DC cells were cultured for six days; thereafter, the cells were blown, and immature DC cells were separated. Centrifugation was performed, and cells were then re-suspended in medium 1640.
3. Mitomycin was added (a final concentration thereof was controlled at 50 µg/mL); the medium 1640 was then treated in water bath at a temperature of 37°C for 15 min. After that, medium 1640 was then washed once and cells were counted.
4. Spleens of C57bl/6 mice were collected, and lymphocytes were separated by using lymphocyte separation medium and were counted.
5. Lymphocytes of spleen of C57bl/6 mice were labeled with CFSE.
6. DC cells from Bal b/c mice and lymphocytes of spleen of C57bl/6 mice were performed mixed lymphocyte culture. Groups was divided as follows:
   Blank group: neither CPG nor TJ-M2010 was added in the medium during the mixed lymphocyte culture;
   Control group: CPG but no TJ-M2010 was added in the medium during the mixed lymphocyte culture;
   Experimental group 1: both CPG and TJ-M2010 were added in the medium during the mixed lymphocyte culture, a dose of TJ-M2010 was 10 µM;
   Experimental group 2: both CPG and TJ-M2010 were added in the medium during the mixed lymphocyte culture, a dose of TJ-M2010 was 20 µM; and
   Experimental group 3: both CPG and TJ-M2010 were added in the medium during the mixed lymphocyte culture, a dose of TJ-M2010 was 40 µM.

1. The mixed lymphocyte culture was cultured until a third day, cells were collected, and lymphocytes proliferation of C57bl/6 mice was detected by using flow cytometry.
   Results of the flow cytometry were shown in FIG. **7****.**
   From the results, it was known that with the increase of the dose of TJ-M2010, T cells (labeled with CD44 on surfaces thereof) proliferation was lowered.
   The results indicated that TJ-M2010 was able to inhibit the activation of DC cells by weakening the CpG thereby inhibiting the T cells proliferation.
   Real-time quantitative PCR comprised the following steps: 1. Total RNA was extracted by TRIzol method from recipient stimulated by (syngeneic and allogeneic) antigens.
2. Total RNA was allowed to reverse transcript into cDNA, and two steps RT-PCT was performed.
3. Standard curve was charted and a relative levels of IL-1β, TNF-α, and IL-6 were obtained.

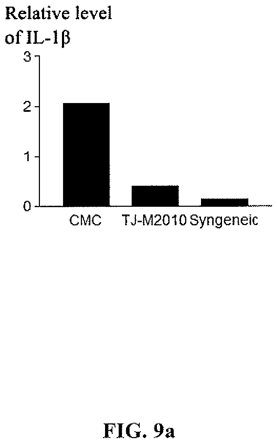

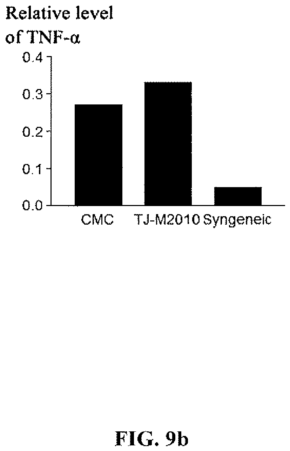

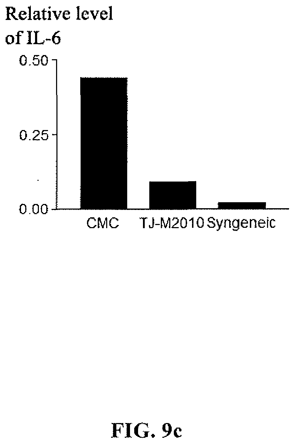

Real-time quantitative PCR analyses of inflammatory factors (IL-1β, TNF-α, and IL-6) in the implant was shown in FIG **9****.**

Results showed that: the level of inflammatory factors in the implant after the heart transplantation in TJ-M2010 group was significantly lower than that of the control group and had statistically significant differences.

From the in vitro experiment, it was known that the inhibitor TJ-M2010 of MyD88 effectively lowered the inflammatory factors in the implant after transplantation by inhibiting the corresponding pathway (levels of IL-1β and IL-6 were significantly lower than that of CMC allogeneic transplantation group), which means that inhibitor TJ-M2010 of MyD88 had a close relationship with the inflammatory factors of the implant, so that it was possible to be an effective method to treat various inflammation diseases.

In vivo experiment: the bolcking of MyD88 pathway to lower the air-tube inflammatory response

The in vivo experiment comprised the following steps:
1. Animals were divided into the following groups: C57bl/6(B6) NaCl group (administered in a dosage of 200 mL by nose drop), C57bl/6(B6)BLM group, TJ-M2010BLM group (TJ-M2010 dissoved in 0.5% CMC was respectively intraperitoneally injected on a 0-3^{th} day, a 5^{th} day, a 7^{th} day, a 9^{th} day, a 11^{th} day, a 13^{th} day, a 15^{th} day, a dose thereof was 150 mg/kg/d).
2. Pneumonia Model building by nose drop of BLM (bleomycin): Air-tube anesthesia was performed by using 40 µL of Ketamine xylazine, a BLM sulfate was administered by nose drop (a dose thereof was 300 µg or 15 mg/kg).
3. Cells and cytokines were collected by bronchoalveolar lavage fluid (BAL): the air-duct was opened and inserted with a plastic sleeve to perform lavage by 0.3 mL of PBS at a temperature of 37°C; after that, lavage solution was extracted (exceeding 95% of the lavage solution was extracted), and the extraction was repeated for 10 times. The lavage solution was divided into two parts: one part was used for cytokines detection (600 g of the lavage solution was centrifuged for 10 min and a supernatant was preserved at a temperature of -80°C for detection), and the other part was used for cells counting (together with 0.4 mL of the lower layer for re-suspension at a temperature of 4°C).
4. Cells and cytokines detection in lung homogenates: after BAL, a whole lung was collected, mashed, and centrifuged. A supernatant was collected and preserved at a temperature of -80°C for MPO detection.
5. Lung MPO activity detection: brine was used to fully lavage the lung through a right heart. The lung was homogenized, and a supernatant was separated. 1 mL of PBS (containing 0.5% of HTAB and 5 mM of EDTA) was added for re-suspension and precipitation. A resulting mixture was centrifuged again, and 50 µL of a supernatant was transferred to a test tube (200 µL of PBS-HTAB-EDTA, 2 mL of HBSS, 100 µL of O-dianisidine dihydrochloride having a concentration ofl.25 mg/mL, 100 µL of 0.05% H₂O₂) and maintained for 15 min. The test tube was then transferred to a vortex tank at the temperature of 37°C, and 100 µl NaN3 1% was used to stop the reaction, and MPO absorbance value at 460 nm was detected.
6. Cells counting: MG-1L was used to stain for 4 min, 95% GS-500 for 8 min, and cells were smeared for counting.
7. Cytokines detection: IL-6 level was detected by ELISA.
8. Statistical analysis: U test and analysis of statistical difference.

FIG. **9** showed that levels of accumulated neutrophils and lymphocytes were decreased during the bronchitis of MYD88-/- mice.

Experimental groups: B6NaCL group, B6BLM group, and TJ-M2010BLM group; each group had 4 mice.

Accumulated neutrophils were significantly decreased in bronchoalveolar of TJ-M2010.

FIG. **9 (a)** showed that total cells on the 1^{st}, 7^{th}, and 11^{th} days, and WT mice group and TJ-2010BLM group had statistical difference.

FIG. **9 (b)** showed that the number of neutrophils in bronchoalveolar of WT mice achieved a peak value, lasted for 7 days, and then returned at the 11^{th} day; while number of neutrophils in bronchoalveolar of the TJ-M2010 group was obviously decreased.

FIG **11** showed that BLM-induced pneumonia symptom was alleviated in the TJ-M2010BLM group, represented as the decrease of the inflammatory cell and inflammatory factors.

FIG. **11 (a)** showed that the level of MPO factors (detected on the 7^{th} day) in the lung tissue was lowered.

FIG. **11 (b)** showed that the level of IL-6 (detected at 24^{th} h) in the lung tissue was lowered.

From experiments results of the accumulation of inflammatory cells and the release of inflammatory factors, it was known that BLM-induced pneumonia symptom was significantly alleviated in the TJ-M2010BLM group, so that the anti-inflammation effect of TJ-M2010 was proved.

The above experiments proved that the blocking of MyD88 was capable of alleviating the inflammatory symptoms, and Inhibitor of MyD88 was applicable in treatment of various chronic inflammatory diseases, such as inflammatory bowel disease and asthma.

### Example 5 Use of inhibitor of MyD88 for treatment of endotoxemia and sepsis

Part one: observation of the influence of Inhibitor of MyD88 on the death rate of mice having endotoxemia disease. The mice were randomly divided into two groups: vehicle control group and TJ-M2010 treatment group, each group had 20 mice. The mice of the TJ-M2010 treatment group were intragastrically administered with TJ-M2010 (0.5% CMC, 25 mg/mL), a dose of TJ-M2010 was controlled 250 mg/kg (200 µL for each). The mice of the vehicle control group were intragastrically administered with 0.5% CMC (200 µL for each). Each mice was administered once a day, and was continuously administered for three days. On a 3^{th} day after the intragastric administration, LPS was intraperitoneal injected, and survival of the mice was observed for every 12 hours, and was continuously observed for three days. Survival curve chart was shown in FIG. **12 (a)****.**

As shown in FIG. **12 (a)****,** the two inhibitors of MyD88 were able to effectively delay the death resulting from endotoxin, and lower the death rate resulting from endotoxin.

Part two: observation of the influence of Inhibitor of MyD88 on the death rate of mice having sepsis disease. The mice were randomly divided into sham operation group, modeling group, and Inhibitor of MyD88 treatment group. Except that the sham operation group was conducted with laparotomy and sutured; both the modeling group and MyD88 inhibitor treatment group were operated with cecal ligation and puncture, and copied sepsis mice modeling. One hour after the surgery, 200 µL of 0.5% CMC or 250 mg/kg (200 µL) of TJ-M2010 were administered every 12 hours, and continuously administered for 4 times. Survival condition of the mice was observed for every 12 hours, and was continuously observed for 72 hours. Survival curve chart was shown in FIG. **12 (b)****.**

As shown in FIG. **12 (b)****,** the inhibitor of MyD88 had an obvious improvement on prolonging the survival duration and lowering the death rate of mice having sepsis disease.

## Claims

1. An aminothiazole-based specific inhibitor of MyD88 (myeloid differentiation protein 88) for use in reducing rejections after an organ transplantation as an immunosuppressive agent and being represented by the following formulas:
1) TJ-M2010-1 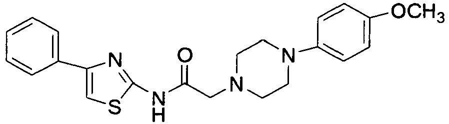 2-(4-(4-methoxyphenyl)piperazin-1-yl)-N-(4-phenyl-thiazole-2-yl) acetamide
2) TJ-M2010-2 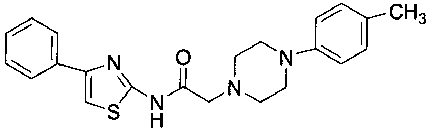 2-(4-(p-tolyl)piperazin-1-yl)-N-(4-phenyl-thiazole-2-yl)acetamide
3) TJ-M2010-3 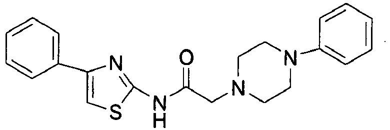 2-(4-phenyl-piperazin-1-yl)-N-(4-phenyl-thiazole-2-yl)acetamide
4) TJ-M2010-4 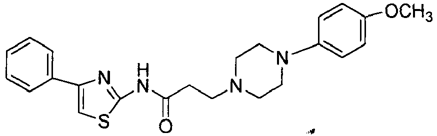 3-(4-(4-methoxyphenyl)piperazin-1-yl)-N-(4-phenyl-thiazole-2-yl)-propionamide

2. An aminothiazole-based specific inhibitor of MyD88 (myeloid differentiation protein 88) for use in the treatment of chronic inflammatory diseases as an anti-inflammatory agent and being represented by the following formulas:
1) TJ-M2010-1 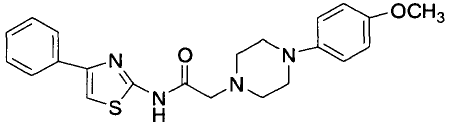 2-(4-(4-methoxyphenyl)piperazin-1-yl)-N-(4-phenyl-thiazole-2-yl) acetamide
2) TJ-M2010-2 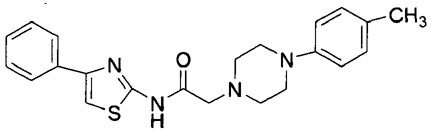 2-(4-(p-tolyl)piperazin-1-yl)-N-(4-phenyl-thiazole-2-yl)acetamide
3) TJ-M2010-3 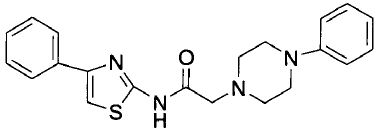 2-(4-phenyl-piperazin-1-yl)-N-(4-phenyl-thiazole-2-yl)acetamide
4) TJ-M2010-4 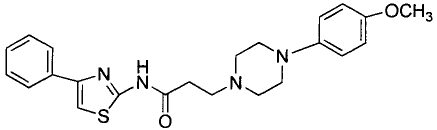 3-(4-(4-methoxyphenyl)piperazin-1-yl)-N-(4-phenyl-thiazole-2-yl)-propionamide

3. An aminothiazole-based specific inhibitor of MyD88 (myeloid differentiation protein 88) for use in the treatment of autoimmune diseases and being represented by the following formulas:
1) TJ-M2010-1 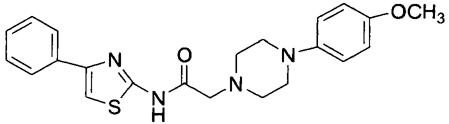 2-(4-(4-methoxyphenyl)piperazin-1-yl)-N-(4-phenyl-thiazole-2-yl) acetamide
2) TJ-M2010-2 2-(4-(p-tolyl)piperazin-1-yl)-N-(4-phenyl-thiazole-2-yl)acetamide
3) TJ-M2010-3 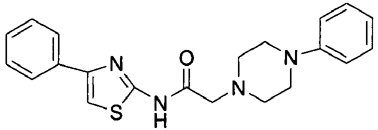 2-(4-phenyl-piperazin-1-yl)-N-(4-phenyl-thiazole-2-yl)acetamide
4) TJ-M2010-4 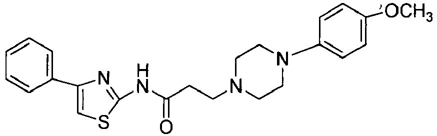 3 - (4 - (4 - methoxyphenyl) piperazin-1 - yl)-N-(4 - phenyl-thiazole-2-yl)-propionamide

4. An aminothiazole-based specific inhibitor of MyD88 (myeloid differentiation protein 88) for use in the treatment of ischemia-reperfusion injuries and being represented by the following formulas:
1) TJ-M2010-1 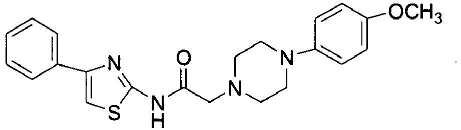 2-(4-(4-methoxyphenyl)piperazin-1-yl)-N-(4-phenyl-thiazole-2-yl) acetamide
2) TJ-M2010-2 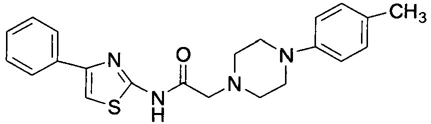 2-(4-(p-tolyl)piperazin-1-yl)-N-(4-phenyl-thiazole-2-yl)acetamide
3) TJ-M2010-3 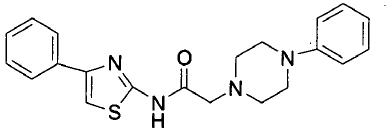 2-(4-phenyl-piperazin-1-yl)-N-(4-phenyl-thiazole-2-yl)acetamide
4) TJ-M2010-4 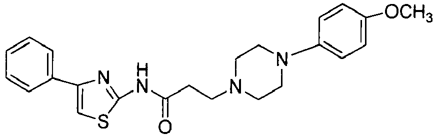 3-(4-(4-methoxyphenyl)piperazin-1-yl)-N-(4-phenyl-thiazole-2-yl)-propionamide

5. An aminothiazole-based specific inhibitor of MyD88 (myeloid differentiation protein 88) for use for prevention of endotoxemia and sepsis and being represented by the following formulas:
1) TJ-M2010-1 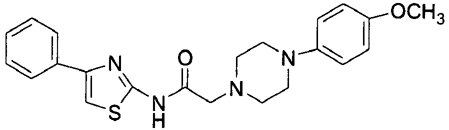 2-(4-(4-methoxyphenyl)piperazin-1-yl)-N-(4-phenyl-thiazole-2-yl) acetamide
2) TJ-M2010-2 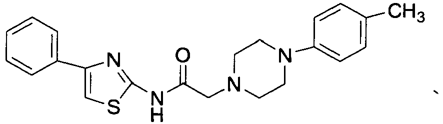 2-(4-(p-tolyl)piperazin-1-yl)-N-(4-phenyl-thiazole-2-yl)acetamide
3) TJ-M2010-3 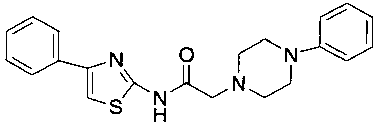 2-(4-phenyl-piperazin-1-yl)-N-(4-phenyl-thiazole-2-yl)acetamide
4) TJ-M2010-4 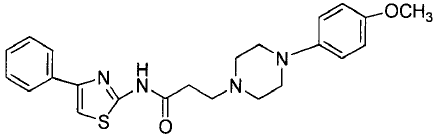 3-(4-(4-methoxyphenyl)piperazin-1-yl)-N-(4-phenyl-thiazole-2-yl)-propionamide

## Patentansprüche

1. Aminothiazol-basierter spezifischer Hemmer von MyD88 (Myeloid-Differenzierungsprotein 88) zur Verwendung zum Vermindern der Abstoßungen nach einer Organtransplantation als ein immunsuppressives Mittel und dargestellt durch die folgenden Formeln:
1) TJ-M2010-1 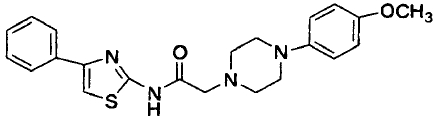 2-(4-(4-Methoxyphenyl)piperazin-1-yl)-N-(4-phenyl-thiazol-2-yl) acetamid
2) TJ-M2010-2 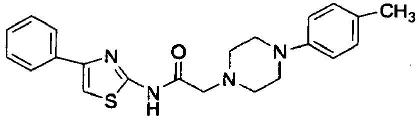 2-(4-(p-Tolyl)piperazin-1-yl)-N-(4-phenyl-thiazol-2-yl) acetamid
3) TJ-M2010-3 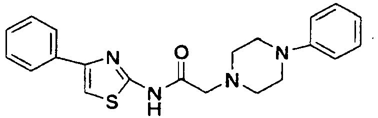 2-(4-Phenyl-piperazin-1-yl)-N-(4-phenyl-thiazol-2-yl)acetamid
4) TJ-M2010-4 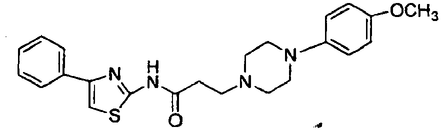 3-(4-(4-Methoxyphenyl)piperazin-1-yl)-N-(4-phenyl-thiazol-2-yl)propionamid

2. Aminothiazol-basierter spezifischer Hemmer von MyD88 (Myeloid-Differenzierungsprotein 88) zur Verwendung zur Behandlung chronischer Entzündungserkrankungen als ein entzündungshemmendes Mittel und dargestellt durch die folgenden Formeln:
1) TJ-M2010-1 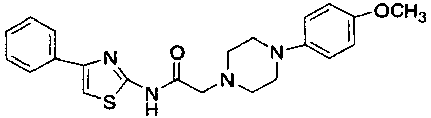 2-(4-(4-Methoxyphenyl)piperazin-1-yl)-N-(4-phenyl-thiazol-2-yl) acetamid
2) TJ-M2010-2 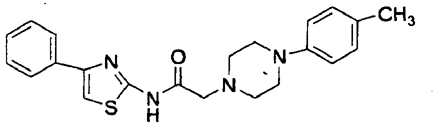 2-(4-(p-Tolyl)piperazin-1-yl)-N-(4-phenyl-thiazol-2-yl) acetamid
3) TJ-M2010-3 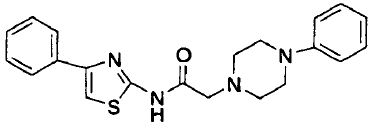 2-(4-Phenyl-piperazin-1-yl)-N-(4-phenyl-thiazol-2-yl) acetamid
4) TJ-M2010-4 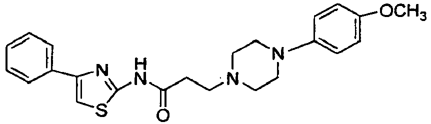 3-(4-(4-Methoxyphenyl)piperazin-1-yl)-N-(4-phenyl-thiazol-2-yl)propionamid

3. Aminothiazol-basierter spezifischer Hemmer von MyD88 (Myeloid-Differenzierungsprotein 88) zur Verwendung zur Behandlung von Autoimmunerkrankungen und dargestellt durch die folgenden Formeln:
1) TJ-M2010-1 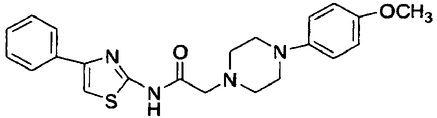 2-(4-(4-Methoxyphenyl)piperazin-1-yl)-N-(4-phenyl-thiazol-2-yl) acetamid
2) TJ-M2010-2
2-(4-(p-Tolyl)piperazin-1-yl)-N-(4-phenyl-thiazol-2-yl) acetamid
3) TJ-M2010-3 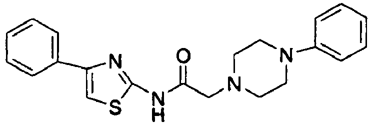 2-(4-Phenyl-piperazin-1-yl)-N-(4-phenyl-thiazol-2-yl) acetamid
4) TJ-M2010-4 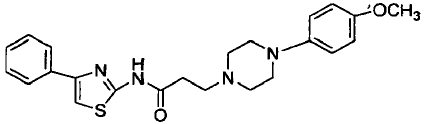 3-(4-(4-Methoxyphenyl)piperazin-1-yl)-N-(4-phenyl-thiazol-2-yl)propionamid

4. Aminothiazol-basierter spezifischer Hemmer von MyD88 (Myeloid-Differenzierungsprotein 88) zur Verwendung zur Behandlung von Ischämie-Reperfusionsschäden und dargestellt durch die folgenden Formeln:
1) TJ-M2010-1 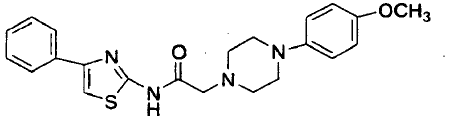 2-(4-(4-Methoxyphenyl)piperazin-1-y1)-N-(4-phenyl-thiazol-2-yl) acetamid
2) TJ-M2010-2 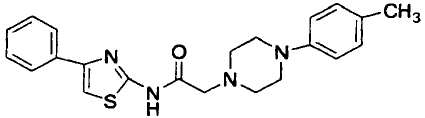 2-(4-(p-Tolyl)piperazin-1-yl)-N-(4-phenyl-thiazol-2-yl) acetamid
3) TJ-M2010-3 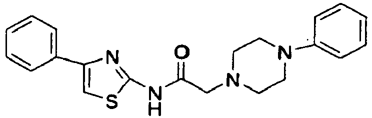 2-(4-Phenyl-piperazin-1-yl)-N-(4-phenyl-thiazol-2-yl) acetamid
4) TJ-M2010-4 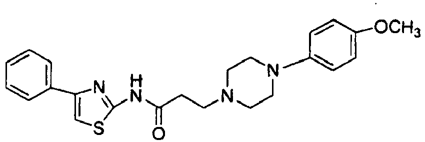 3-(4-(4-Methoxyphenyl)piperazin-1-yl)-N-(4-phenyl-thiazol-2-yl)propionamid

5. Aminothiazol-basierter spezifischer Hemmer von MyD88 (Myeloid-Differenzierungsprotein 88) zur Verwendung zur Vorbeugung gegen Endotoxämie und Sepsis und dargestellt durch die folgenden Formeln:
1) TJ-M2010-1 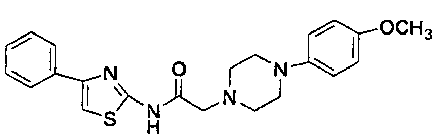 2-(4-(4-Methoxyphenyl)piperazin-1-yl)-N-(4-phenyl-thiazol-2-yl) acetamid
2) TJ-M2010-2 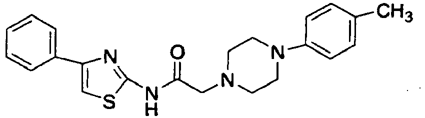 2-(4-(p-Tolyl)piperazin-1-yl)-N-(4-phenyl-thiazol-2-yl) acetamid
3) TJ-M2010-3 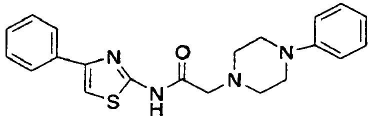 2-(4-Phenyl-piperazin-1-yl)-N-(4-phenyl-thiazol-2-yl) acetamid
4) TJ-M2010-4 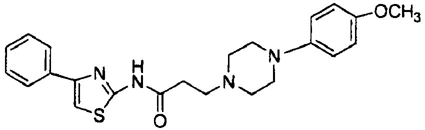 3-(4-(4-Methoxyphenyl)piperazin-1-yl)-N-(4-phenyl-thiazol-2-yl)propionamid

## Revendications

1. Inhibiteur à base d'aminothiazole spécifique de MyD88 (protéine de différentiation de myéloïde 88) pour une utilisation dans la réduction des rejets après une greffe d'organe comme agent immunosuppresseur et étant représenté par les formules suivantes :
1) TJ-M2010-1 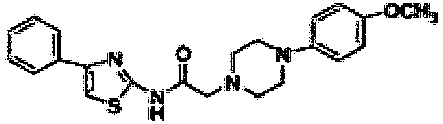 2-(4-(4-méthoxyphényl)pipérazine-1-yl)-N-(4-phényl-thiazole-2-yl) acétamide
2) TJ-M2010-2 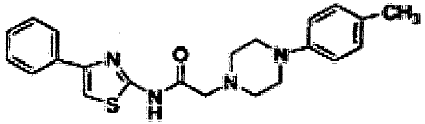 2-(4-(p-tolyl)pipérazine-1-yl)-N-(4-phényl-thiazole-2-yl) acétamide
3) TJ-M2010-3 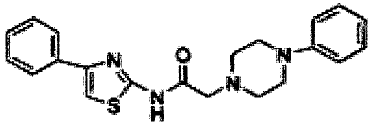 2-(4-phényl-pipérazine-1-yl)-N-(4-phényl-thiazole-2-yl) acétamide
4) TJ-M2010-4 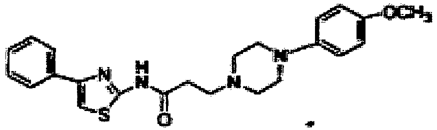 3-(4-(4-méthoxyphényl)pipérazine-1-yl)-N-(4-phényl-thiazole-2-yl)-propionamide

2. Inhibiteur à base d'aminothiazole spécifique de MyD88 (protéine de différentiation de myéloïde 88) pour une utilisation dans le traitement de maladies inflammatoires chroniques comme agent anti-inflammatoire et étant représenté par les formules suivantes :
1) TJ-M2010-1 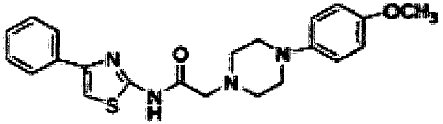 2-(4-(4-méthoxyphényl)pipérazine-1-yl)-N-(4-phényl-thiazole-2-yl) acétamide
2) TJ-M2010-2 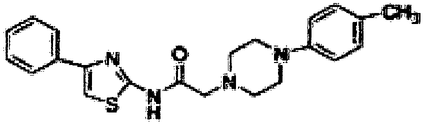 2-(4-(p-tolyl)pipérazine-1-yl)-N-(4-phényl-thiazole-2⁻yl) acétamide
3) TJ-M2010-3 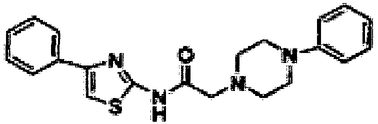 2-(4-phényl-pipérazine-1-yl)-N-(4-phényl-thiazole-2-yl) acétamide
4) TJ-M2010-4 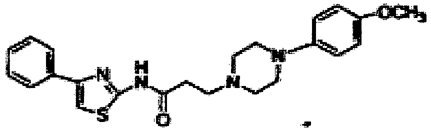 3-(4-(4-méthoxyphényl)pipérazine-1-yl)-N-(4-phényl-thiazole-2-yl)-propionamide

3. Inhibiteur à base d'aminothiazole spécifique de MyD88 (protéine de différentiation de myéloïde 88) pour une utilisation das le traitement de maladies autoimmunes et étant représenté par les formules suivantes :
1) TJ-M2010-1 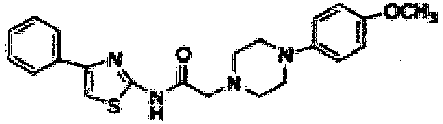 2-(4-(4-méthoxyphényl)pipérazine-1-yl)-N-(4-phényl-thiazole-2-yl) acétamide
2) TJ-M2010-2
2-(4-(p-tolyl)pipérazine-1-yl)-N-(4-phényl-thiazole-2-yl) acétamide
3) TJ-M2010-3 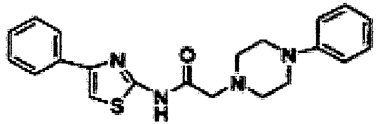 2-(4-phényl-pipérazine-1-yl)-N-(4-phényl-thiazole-2-yl) acétamide
4) TJ-M2010-4 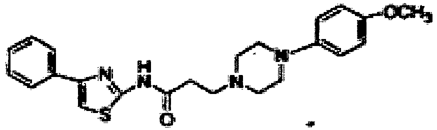 3-(4-(4-méthoxyphényl)pipérazine-1-yl)-N-(4-phényl-thiazole-2-yl)-propionamide

4. Inhibiteur à base d'aminothiazole spécifique de MyD88 (protéine de différentiation de myéloïde 88) pour une utilisation dans le traitement des lésions d'ischémie-reperfusion et étant représenté par les formules suivantes :
1) TJ-M2010-1 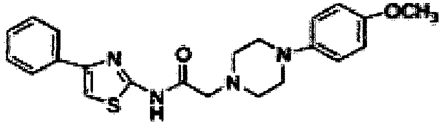 2-(4-(4-méthoxyphényl)pipérazine-1-yl)-N-(4-phényl-thiazole-2-yl) acétamide
2) TJ-M2010-2 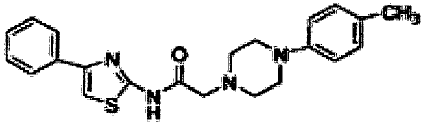 2-(4-(p-tolyl)pipérazine-1-yl)-N-(4-phényl-thiazole-2-yl) acétamide
3) TJ-M2010-3 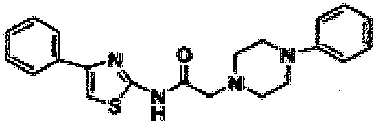 2-(4-phényl-pipérazine-1-yl)-N-(4-phényl-thiazole-2-yl) acétamide
4) TJ-M2010-4 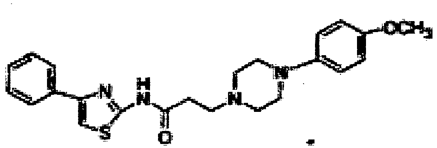 3-(4-(4-méthoxyphényl)pipérazine-1-yl)-N-(4-phényl-thiazole-2-yl)-propionamide

5. Inhibiteur à base d'aminothiazole spécifique de MyD88 (protéine de différentiation de myéloïde 88) pour une utilisation pour la prévention de l'endotoxémie et de la sepsie et étant représenté par les formules suivantes :
1) TJ-M2010-1 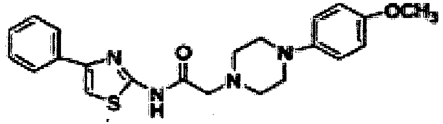 2-(4-(4-méthoxyphényl)pipérazine-1-yl)-N-(4-phényl-thiazole-2-yl) acétamide
2) TJ-M2010-2 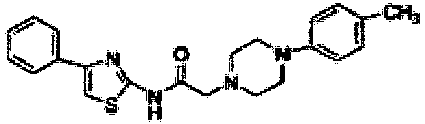 2-(4-(p-tolyl)pipérazine-1-yl)-N-(4-phényl-thiazole-2-yl) acétamide
3) TJ-M2010-3 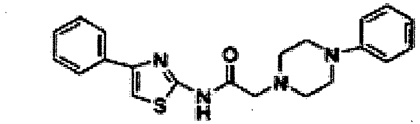 2-(4-phényl-pipérazine-1-yl)-N-(4-phényl-thiazole-2-yl) acétamide
4) TJ-M2010-4 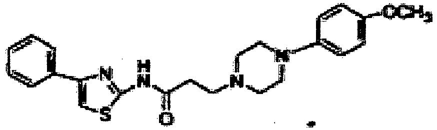 3-(4-(4-méthoxyphényl)pipérazine-1-yl)-N-(4-phényl-thiazole-2-yl)-propionamide
